**Europäisches Patentamt**

**European Patent Office**

**Office européen des brevets**

(19)

(11) Publication number: **0 123 164**
**A1**

(12) **EUROPEAN PATENT APPLICATION**

(21) Application number: **84103411.9**

(22) Date of filing: **28.03.84**

(51) Int. Cl.³: **A 61 M 5/28**
**B 65 D 83/00**

(30) Priority: **30.03.83 SE 8301775**

(43) Date of publication of application:
**31.10.84 Bulletin 84/44**

(84) Designated Contracting States:
**AT BE CH DE FR GB IT LI LU NL**

(71) Applicant: **Holasek, Jiri**
**Sandgatan 8**
**S-331 00 Värnamo(SE)**

(72) Inventor: **Holasek, Jiri**
**Sandgatan 8**
**S-331 00 Värnamo(SE)**

(74) Representative: **Asketorp, Göran P. et al,**
**Lars Holmqvist Patentbyra AB Box 4289**
**S-203 14 Malmö 4(SE)**

(54) Package.

(57) The present invention discloses an expendable package (1) for administration of fluid or semi-fluid products (7). The package comprises a nozzle portion (3) and an ampoule portion (2). The ampoule portion consists of an outer (4) and an inner (5) container, between which there is a sealed space (6) filled with a gas or liquid medium.

*Fig. 2*

EP 0 123 164 A1

0123164

1

# PACKAGE

The present invention relates to an expendable package for storing and administration of a fluid or semi-fluid product comprising a nozzle portion and a deformable ampoule portion, the ampoule portion comprising an outer and an inner container and a sealed space therebetween filled with a gas or a liquid.

A number of expendable packages especially for pharmaceutical preparations are previously known.

A common type of packages for administration of e.g. eye drops and similar consists of a simple soft or semi-soft container which is pressed together when used. A drawback with this kind of container is the fact that it is almost impossible to achieve 100 percent emptying of the container. Furthermore, to elderly persons and rheumatics, etc. it is very difficult to press with a power sufficient to achieve effective emptying.

For injection of different pharmaceutical preparations there are expendable syringes where the content by means of a piston is pressed out through the cannula. However, the handling is rather complicated and failures do occur. Another problem with these expendable syringes is the fact that they can be re-used.

For this reason syringes are often desirable objects to abusers of narcotics. Moreover, the infection risk at re-use is substantial.

In order to eliminate the above-mentioned problem, especially when experienced by elderly persons and other persons having weak joints, to empty the container and to achieve 100 percent emptying of the container several different kinds of ampoules have been suggested; ampoules with decreasing cross-section towards the nozzle portion, ampoules in the shapes of bellows and concertinas. However, in practice none of these has turned out to dissolve the above mentioned problem, and concerning the ampoules in the shapes of bellows and concertinas the "death-space" is important.

Another disadvantage with the existing expendable packages is that the space occupied by the package is the same before and after the emptying. With regard to the amount which is used today within the medical attendance it must be considered that the discarded volume is a problem.

The object of the present invention is to provide a package or container which by simple and fast coupling to an emptying device, e.g. a syringe gives a unit which is simple to handle and inexpensive.

The object of the present invention is also to provide an expendable package which is constructed in such a way that it is emptied to 100 percent, i.e. gives possibility to an exact dosage.

Another object of the present invention is to provide a package, which can be handled and emptied to 100 percent, also by feeble persons.

Still another object of the present invention is to provide a package which cannot be re-used and thus not abused.

These objects are achieved by means of an expendable package for administration of a fluid or semi-fluid product, preferably a pharmaceutical preparation, comprising a nozzle portion and a deformable ampoule portion, whereby the ampoule portion comprises an outer container and an inner container and a sealed space between the outer and the inner container, the inner container including the fluid or semi-fluid product to be administrated and the sealed space between the outer and the inner container including a pressure transmitting gas or liquid medium, the outer and the inner container being made of elastic material.

Due to the enclosed pressure transmitting gas or liquid (fluid or semi-fluid) or combinations thereof, a complete emptying of the pharmaceutical preparation or other product comprised in the ampoule portion is obtained. According to hydrostatic principles an external pressure (e.g. finger pressure) which is effecting the outer container will be distributed all over the gas or liquid encompassed in the outer container. Since no friction occurs the pressure on the inner container is perpendicular to each elementary surface element and the inner container will be completely emptied, independent of the shape. The inner container experiences a pressure from all directions, independent of the shape of the fingers and independent of the spot of the outer container onto which the pressure is applied. The inner container should be made sufficiently soft in order that persons with reduced strength in their fingers, like elderly persons and rheumatics, are able to squeeze out the content completely. This gives a very careful dosage, which is extremely important in the case of pharmaceutical preparations.

Furthermore, by the pressure exerted by the encompassed gas or liquid medium a re-use is made impossible. When carefully emptied, the inner container remains compressed. The ampoule cannot be re--used by e.g. abusers of narcotics and furthermore the originality of the content is secured.

By the fact that the ampoule portion remains compressed after the emptying, the package is space-saving.

The ampoule can also be destroyed by puncturing the container.

This double container gives a double protection for the content. Even if the outer container is destroyed the pharmaceutical preparation can be administrated, however under changed conditions.

Furthermore, the package according to the present invention is inexpensive to manufacture, easy to handle, not crushable and light.

The package according to the present invention can be used as applicator per se, of the type glass dropper, or be connected to a cannula to be used as a syringe etc.

From the present invention a plastically soft container is provided which is less sensible to destruction during transport etc. than a glass ampoule, which is especially important within the acute medical attendance. Instead of a glass ampoule, a syringe and an extraction cannula and work during antiseptic conditions the present invention provides a pre-filled container which is uncrushable and at the same time functions as a syringe.

Instead of manual emptying the package can be connected to a motor device for automatical emptying.

A more detailed description of the invention is given below with reference to the drawings, where

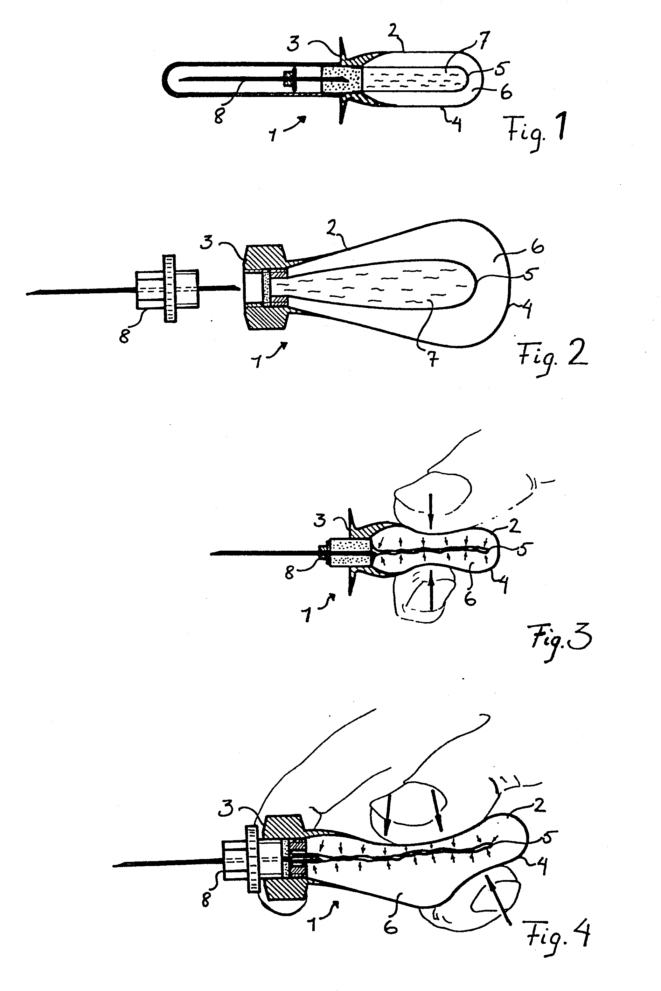

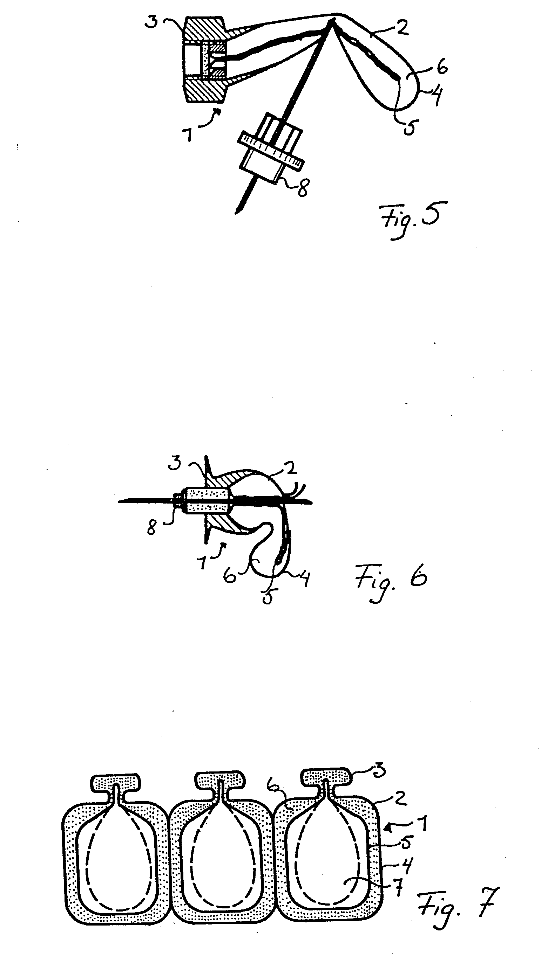

Figs. 1 and 2 disclose different embodiments of filled packages according to the invention comprising a cannula portion; Figs. 3 and 4 disclose the emptying of the packages according to Figs. 1 and 2, respectively; Figs. 5 and 6 disclose two different ways to consciously destroy a package according to the present invention; and Fig. 7 discloses an additional embodiment of the package according to the present invention.

With reference to Figs. 1 and 2 a package 1 is shown comprising a nozzle portion or discharge portion 3 and an ampoule portion 2. The ampoule portion consists in its turn of an outer container 4 and an inner container 5. Between these two containers is a sealed space 6 filled with a gas or liquid medium, preferably air but also any other gas, any liquid, fluid or semi-fluid, or a combination hereof is useful. In the inner container the product 7, fluid or semi-fluid, which is to be administrated, is positioned. According to these two embodiments a cannula portion 8 is connected to the ampoule portion, whereupon all of the unit functions as an injection syringe.

Figs. 3 and 4 disclose how a person by means of an outer pressure (big arrows) presses together the inner container and how this pressure is distributed evenly above and perpendicular against the inner container (small arrows).

Figs. 5 and 6 show how a person consciously can destroy the ampoule in order to further avoid re-use thereof.

Finally, Fig. 7 further shows an embodiment of the package according to the invention in the shape of a multi-package unit. The nozzle portion 3 is here constructed in one piece with the ampoule portion 2, preferably of plastic, and intended to be torn off.

The package according to the present invention is partly intended to be used as an applicator, e.g. as glass dropper, refilling bottle etc., partly to be connected to an applicator portion, as e.g. a cannula.

The package can be sealed in many different, known ways; protected cannula portion, cork, plug, welding, Luer-Lock mounting, different intermediate pieces, etc.

Even if the package according to the invention is especially suitable for pharmaceutical preparations, it is a matter of course that an amount of other fluid or semi-fluid products can be administrated by means thereof, e.g. articles of food, schampoo, soap, etc.

The ampoule portion is preferably made of plastic and in those cases where a severe mechanical wear could be expected the outer container can be made stronger than the inner container without rendering the emptying more difficult.

The package according to the present invention can be modified in many ways, which are well-known to a person skilled in the art and the invention is not limited by the embodiments shown but only with respect to the accompanying claims.

6

## CLAIMS

1. Expendable package (1) for administration of a fluid or semi-fluid product (7) comprising a nozzle portion (3) and a deformable ampoule portion (2), c h a r a c t e r i z e d  in that the ampoule portion (2) comprises an outer container (4) and an inner container (5), and a sealed space (6) between the outer and the inner container, the inner container including the fluid or semi-fluid product to be administrated and the sealed space between the outer and the inner container including a pressure transmitting gas or liquid medium, the outer (4) and the inner container (5) being made of elastic material.

2. Package according to claim 1, c h a r a c t e r i z e d  in that the medium in the space (6) between the outer (4) and the inner container (5) is air.

3. Package according to claim 2, c h a r a c t e r i z e d in that the elasticity for the outer (4) container is different from that of the inner container (5).

4. Package according to anyone of previous claims, c h a r a c-t e r i z e d  in that the nozzle portion (3) is constructed in one piece with the ampoule portion (2).

0123164

1/2

Fig. 1

Fig. 2

Fig. 3

Fig. 4

0123164

Fig. 5

Fig. 6

Fig. 7

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | US-A-3 244 173 (BERG) <br> * Column 1, line 54 - column 2, line 47, figures 1-4 * | 1 | A 61 M 5/28 <br> B 65 D 83/00 |
| Y | | 2-4 | |
| Y | FR-A-1 316 596 (BOUET) <br> * Page 2, left-hand column, line 56 - right-hand column, line 2; figure 3 * | 2-4 | |
| A | US-A-2 564 359 (FULLER) <br> * Column 5, lines 17-34; figures 1-3 * | 1,2,4 | |
| A | * Column 3, lines 14-33 * | 3 | TECHNICAL FIELDS SEARCHED (Int. Cl. ³) |
| A | US-A-2 911 972 (ELINGER) | | A 61 M <br> B 65 D |

The present search report has been drawn up for all claims

| Place of search <br> THE HAGUE | Date of completion of the search <br> 09-07-1984 | Examiner <br> KNAUER F.E. |
|---|---|---|